**Europäisches Patentamt**

**European Patent Office**

**Office européen des brevets**

(19)

(11) Veröffentlichungsnummer: **0 114 281**
A2

(12) # EUROPÄISCHE PATENTANMELDUNG

(21) Anmeldenummer: **83112348.4**

(51) Int. Cl.³: **B 01 D 39/18**

(22) Anmeldetag: **08.12.83**

(30) Priorität: **27.12.82 DE 3248214**

(43) Veröffentlichungstag der Anmeldung:
**01.08.84 Patentblatt 84/31**

(84) Benannte Vertragsstaaten:
**AT CH FR GB IT LI SE**

(71) Anmelder: **RHODIA AG**
**Engesserstrasse 8**
**D-7800 Freiburg(DE)**

(72) Erfinder: **Schlüter, Gert**
**Im Höfle 22**
**D-7803 Gundelfingen(DE)**

(72) Erfinder: **Albrecht, Erwin**
**Matthias-Blank-Strasse 48**
**D-7800 Freiburg(DE)**

(54) Vorrichtung zur Gewinnung von Zellmaterial aus Körperflüssigkeiten.

(57) Vorrichtung zur Gewinnung von Zellmaterial aus Körperflüssigkeiten, bestehend aus einem Filter mit einem in einer Schicht oder in mehreren Schichten angeordneten, in zellfixierenden Flüssigkeiten wie Isopropanol nicht löslichen Filtermaterial. Das Filtermaterial besteht aus einem einzigen texturierten oder gekräuselten Filamentgarn. Vorzugsweise bestehen die Filamente des Filamentgarns aus synthetischen Polymeren.

Im Gegensatz zu einer bekannten Vorrichtung, im wesentlichen bestehend aus einer Filterpatrone, die als Filtermaterial synthetische, in Alkohol oder in einem Alkohol-Wasser-Gemisch auflösbare Fasern enthält, wird beim Einsatz der erfindungsgemäßen Vorrichtung die gewonnene Zellprobe nicht mit Partikeln des Filtermaterials durchsetzt. Außerdem erreicht man mit der Erfindung sehr niedrige Zellverluste, und es treten keine Zellschädigungen auf.

Körperflüssigkeiten, aus denen mit der Vorrichtung gemäß der Erfindung Zellmaterials gewonnen werden kann, sind z.B. Urin, Liquor, Pleuraergüsse oder Ascites.

## Vorrichtung zur Gewinnung von Zellmaterial aus Körperflüssigkeiten

Die Erfindung betrifft eine Vorrichtung zur Gewinnung von Zellmaterial aus Körperflüssigkeiten, bestehend aus einem Filter mit einem in einer Schicht oder in mehreren Schichten angeordneten, in zellfixierenden Flüssigkeiten nicht löslichen Filtermaterial.

Um im menschlichen Organismus Krankheiten und insbesondere maligne Entartungen im Frühstadium erkennen zu können, nimmt die Diagnostik zellulärer Bestandteile aus Organen einen hohen Stellenwert ein.
Die Zellgewinnung dafür erfolgt

- von Organoberflächen mittels Abstrichverfahren,

- aus tieferen Organregionen mittels Biopsie- und Punktionsverfahren,

- aus Körperflüssigkeiten, nämlich physiologisch vorhandenen Körperflüssigkeiten, wie Urin oder Liquor, oder durch pathologische Prozesse gebildeten Körperflüssigkeiten, wie Pleuraergüssen und Ascites, durch Zentrifugation oder durch Filtration mit Hilfe von Membranfiltern.

Die bekannten Verfahren und Vorrichtungen zur Gewinnung von Zellen aus Körperflüssigkeiten haben den Nachteil, daß entweder eine Fixierung der Zellen in der entnommenen oder abgeschiedenen Körperflüssigkeit nicht sofort, sondern erst nach der Aufarbeitung erfolgt oder beim Aufbereitungs- oder Wiedergewinnungsprozeß zusätzliche Zellschädigungen erzeugt werden.

Ganz gravierend ist die hohe Verlustrate bei der Zentrifugation, da bei ihr bis zu 40 % des in der Körperflüssigkeit vorhandenen Zellmaterials durch Adhäsion an Wandungen von Zentrifugiergläsern etc. verloren gehen können. Beim Filtrieren mit Hilfe von Membranfiltern sind in der Regel Saug- oder Druckprozesse erforderlich, die an empfindlichen oder vorgeschädigten Zellen zusätzliche Artefakte erzeugen können. Ein weiterer Nachteil ist dabei, daß z.B. bei Urinproben praktisch nie die Gesamtflüssigkeit (Gesamtmiktion) wegen zu hohem Aufwand aufgearbeitet werden kann. Das bedeutet, daß nur ein Teil der Flüssigkeit, in der Regel bis zu 20 ml, filtriert wird, was bei zellarmen Proben stets zu einer Unterbelegung auf dem Objektträger führt.

Aus der deutschen Offenlegungsschrift Nr. 31 08 133 sind ein Verfahren und eine Vorrichtung bekannt, mit dem solche Nachteile vermieden werden sollen.

Das bekannte Verfahren zur Untersuchung von Urin auf partikuläre Bestandteile, insbesondere auf Zellen, besteht darin, daß der Urin unmittelbar nach der Abscheidung oder Entnahme durch ein Filter aus synthetischen, in Alkohol oder in einem Alkohol-Wasser-Gemisch auflösbaren Fasern geleitet wird, daß anschließend die Fasern in einem zellfixierenden Lösungsmittel aufgelöst werden und daß die Probe, d.h. die beim Filtrieren aufgefangenen Zellen, in diesem Lösungsmittel bis zur Untersuchung aufbewahrt wird. Die bekannte Vorrichtung besteht im wesentlichen aus einer Filterpatrone, die als Filtermaterial synthetische, in Alkohol oder in einem Alkohol-Wasser-Gemisch auflösbare Fasern enthält.

Zwar hat eine solche mit Fasern gestopfte Filterpatrone eine gute Filterwirkung, nachteilig ist jedoch, daß beim Einsatz von beispielsweise Äthylcellulosefasern als Filtermaterial für die Filterpatrone diese Fasern nicht vollständig gelöst werden, so daß die Zellprobe mit einem erheblichen Anteil an nicht gelösten Äthylcellulosepartikeln durchsetzt ist, die sich im Objektträgerpräparat nach der für die mikroskopische Untersuchung erforderlichen Anfärbung der Zellen als homogener diffuser Untergrund darstellen.

Eine Reduzierung der nicht gelösten Äthylcellulosepartikel in der Zellprobe ist nur durch wiederholte Waschungen in einer großen Menge hochprozentigen Alkohols möglich, wobei zusätzlich noch Zentrifugationsschritte erforderlich sind.

Ferner ist es bekannt, Zellmaterial aus Körperflüssigkeiten durch Filtration mit Hilfe von Vliesfiltern zu gewinnen.

Solche Vliesfilter haben zwar auch eine gute Filterwirkung, jedoch ist bei ihnen die Wiedergewinnung des Zellmaterials aus dem Vlies sehr schwierig, so daß große Zellverluste auftreten.

Filtergewebe haben wiederum für die Gewinnung von Zellmaterial aus Körperflüssigkeiten den Nachteil, daß sie eine schlechte Filtrationswirksamkeit haben, so daß zuviel Zellmaterial durch das Filtergewebe hindurchläuft.

Der Erfindung liegt die Aufgabe zugrunde, eine Vorrichtung zur Gewinnung von Zellmaterial aus Körperflüssigkeiten zur Verfügung zu stellen, mit welcher Vorrichtung die beschriebenen Nachteile der bekannten Vorrichtungen vermieden werden.

Diese Aufgabe wird bei einer Vorrichtung der eingangs genannten Art zur Gewinnung von Zellmaterial aus Körperflüssigkeiten durch die im Kennzeichen des Anspruchs 1 angegebenen Merkmale gelöst.

Eine vorteilhafte Ausführungsform der erfindungsgemäßen Vorrichtung ist im Anspruch 2 angegeben.

Für die Zwecke der Erfindung eignen sich als Filamente für das Filamentgarn beispielsweise solche aus

- Polyolefin, wie Polypropylen,

- Polyester, wie Polyäthylenterephthalat und/oder

- Polyamid, wie Polyamid -6,6.

Für die erfindungsgemäße Vorrichtung kommen als Filamentgarne solche in Betracht, die durch Texturieren oder Kräuseln, beispielsweise durch Falschdralltexturieren, Blasdüsenkräuseln oder Stauchkammerkräuseln hergestellt worden sind.

Die Erfindung wird nachstehend anhand eines Beispiels näher erläutert.

<u>Beispiel</u>

Zur Herstellung der erfindungsgemäßen Vorrichtung wurde von einem zylindrischen Röhrchen aus Polypropylen ausgegangen. Das Röhrchen hatte eine Länge von 40 mm und einen Innendurchmesser von 7 mm.

Auf der einen Seite war das Röhrchen mit einem festsitzenden zylindrischen Innenring von 5 mm Länge und einem Innendurchmesser von 4,5 mm versehen.

- 5 -

Das Röhrchen wurde auf der anderen Seite innen mit einem Stöpsel aus einem Metallgewebe mit 0,35 mm Maschenweite versehen. Der Stöpsel ragte 10 mm in das Röhrchen hinein und hatte einen festen Sitz im Röhrchen. Das Röhrchen wurde sodann von der mit dem Stöpsel versehenen Seite her 5 mm tief in einen Vakuumschlauch, der mit einer Vakuumpumpe verbunden war, wandbündig festsitzend eingeschoben. Auf der mit dem zylindrischen Innenring versehenen Seite des Röhrchens wurde eine Einsaugdüse, bestehend aus einem zylindrischen Metallrohr mit einer Länge von 100 mm und einem Innendurchmesser von 1,5 mm, aufgesetzt. Das Röhrchen und die Einsaugdüse waren durch eine sowohl Röhrchen als auch Einsaugdüse teilweise überlappende Kunststoffmuffe dicht miteinander verbunden.

Durch die Vakuumpumpe wurde dann ein Unterdruck im Röhrchen von 0,82 bar (= 8200 N/m$^2$) erzeugt. An die freie Öffnung der Einsaugdüse wurde danach der Anfang eines von einer Spule abgezogenen und über eine regelbare Dosiervorrichtung geleiteten Filamentgarns angelegt. Durch den Unterdruck wurde das Filamentgarn in einer Dosierung von 50 mg durch die Einsaugdüse in das Röhrchen hineingesaugt und auf den Stöpsel aus Metallgewebe in mehreren Schichten abgelegt. Danach wurde das Röhrchen vom Vakuumschlauch und von der Einsaugdüse getrennt und der Stöpsel aus Metallgewebe aus dem Röhrchen entfernt.

Anstelle des Stöpsels aus Metallgewebe wurde dann eine zylindrische Hülse aus Polypropylen mit einer Länge von 27 mm, einem Innendurchmesser von 6 mm und einem solchen Außendurchmesser, daß diese Hülse in das Röhrchen hineingeschoben werden konnte und einen festen Sitz im Röhrchen hatte, über eine Länge von 27 mm in das Röhrchen hineingeschoben. Die zylindrische Hülse hatte einen außen geriffelten hohlzylindrischen Aufsatz mit einem Außendurchmesser von 10 mm, so daß die eine Kante dieses Aufsatzes auf der Kante des Röhrchens auflag. Der Innendurchmesser des Aufsatzes entsprach dem der Hülse.

Durch den Innenring und die zylindrische Hülse wurde das im Röhrchen angelagerte Filamentgarn auf eine Länge von 8 mm zusammengedrückt.

Das Filamentgarn war über diese Länge von 8 mm im Röhrchen homogen verteilt.

Als Filamentgarn wurde ein falschdralltexturiertes Polyäthylenterephthalatfilamentgarn 50 dtex f 22 x 1 S, dessen Filamente einen trilobalen Querschnitt aufwiesen, eingesetzt. Das Filamentgarn wies folgende Kräuselwerte nach DIN 53840 auf:

Kräuselelastizität : 36 %

Kräuselbeständigkeit:: 77 %


Das Röhrchen wurde über ein Schlauchzwischenstück mit einem Laborglastrichter verbunden.

Danach wurde eine Zellen enthaltende Urinprobe einer Gesamtmiktion über den Trichter durch das Röhrchen geleitet. Nachdem der Urin vollständig durch das Röhrchen gelaufen war, wurde das Röhrchen vom Schlauchzwischenstück abgekoppelt und die zylindrische Hülse aus dem Röhrchen entfernt. Danach wurde das Röhrchen auf der mit dem zylindrischen Innenring versehenen Seite mit einer Verschlußkappe verschlossen. Das Röhrchen wurde mit seiner offenen Seite paßdicht mit einem zylindrischen rohrförmigen Behälter mit einem Innendurchmesser von 12 mm und einer Länge von 80 mm verbunden. Der Behälter war auf der gegenüberliegenden Seite mit einem Stopfen verschlossen und enthielt 5 ml 30 % - iges Isopropanol. Behälter und mit ihm verbundenes Röhrchen wurden sodann 10 Sekunden lang geschüttelt. Bei diesem Vorgang wurde das Filamentgarn mit Isopropanol durchtränkt. Durch den Einfluß des Isopropanols auf das Filamentgarn sowie durch den Schüttelvorgang bogen sich die Kräuselbögen der Filamente des Filamentgarns auf, wodurch sich das Filamentgarn aufbauschte und sich aus seiner zusammengepreßten Lage löste. Das Filamentgarn verteilte sich deshalb über das gesamte Volumen des Behälters. Dabei ging das dem Filamentgarn durch den Filtriervorgang anhaftende Zellmaterial in das Isopropanol über und wurde dabei gleichzeitig fixiert. Um das Filamentgarn von der Zellsuspension zu trennen, wurde zunächst das Röhrchen vom Behälter abgekoppelt, wobei der Behälter mit dem Stopfen nach unten senkrecht gehalten wurde. Danach wurde mittels einer anatomischen Pinzette oder mit Hilfe eines an seinem Ende angerauhten Stäbchens das Filamentgarn erfaßt und mittels Drehbewegungen und gleichzeitigen Andrückens gegen die Behälterinnenwand oberhalb der Flüssigkeitsoberfläche ausgepreßt, wodurch das restliche Isopropanol und auch noch anhaftende Zellen weitgehend vom Filamentgarn getrennt wurden. Sodann wurde der Behälter in Ruhelage senkrecht mit dem Stopfen nach unten aufgestellt, wodurch das in dem Isopropanol vorhandene Zellmaterial zum Sedimentieren gebracht wurde. Nach dem abgeschlossenen Sedimentationsvorgang wurde das überstehende Isopropanol abdekantiert. Das Zellsediment wurde entweder mittels einer Pipette oder einer Record-Injektionsspritze aufgenommen und auf Objektträger übertragen. Es wurde festgestellt, daß aus der Urinprobe auf diese Weise 95 % der in ihr vorhandenen Zellen mit einem Durchmesser ab 7 µm unbeschädigt gewonnen werden konnten.

Mit einem entsprechenden, zum Vergleich herangezogenen Vliesfilter aus gekräuselten Cellulose-2,5-Acetatfasern konnten aus der gleichen Urinprobe lediglich 60 % der Zellen mit einem Durchmesser ab 7 µm gewonnen werden.

Die Erfindung weist folgende Vorteile auf:

Beim Einsatz der erfindungsgemäßen Vorrichtung sind keine aufwendigen Zentrifugationsschritte notwendig.

Mit der erfindungsgemäßen Vorrichtung treten keine Zellschädigungen auf; außerdem vermeidet man größere Zellverluste.

Beim Einsatz der Vorrichtung gemäß der Erfindung wird die gewonnene Zellprobe nicht mit Bruchstücken oder Rückständen des Filtermaterials durchsetzt.

Die erfindungsgemäße Vorrichtung erlaubt die Fixierung der gewonnenen Zellen sofort nach der Filtration.

Mit der erfindungsgemäßen Vorrichtung wird eine gleich gute Filterwirkung wie mit einem Vliesfilter erreicht, jedoch wesentlich mehr Zellmaterial gewonnen, und zwar durch das Aufbauschen des Filamentgarns in der zellfixierenden Flüssigkeit.

0.1.1.4.2.8.1

## Patentansprüche

1. Vorrichtung zur Gewinnung von Zellmaterial aus Körperflüssigkeiten, bestehend aus einem Filter mit einem in einer Schicht oder in mehreren Schichten angeordneten, in zellfixierenden Flüssigkeiten nicht löslichen Filtermaterial, dadurch gekennzeichnet, daß das Filtermaterial aus einem einzigen texturierten oder gekräuselten Filamentgarn besteht.

2. Vorrichtung nach Anspruch 1, dadurch gekennzeichnet, daß die Filamente des Filamentgarns aus synthetischen Polymeren bestehen.